# EUROPEAN PATENT APPLICATION

(11) **EP 4 656 137 A1**
(43) Date of publication of application: **03.12.2025**
(21) Application number: 24770454.7
(22) Date of filing: 21.02.2024
(51) Int. Cl.: A61B 5/276, A61B 5/256, A61B 5/28

(54) **BIOLOGICAL SIGNAL MEASUREMENT DEVICE AND METHOD FOR CONTROLLING BIOLOGICAL SIGNAL MEASUREMENT DEVICE**

(30) Priority: 15.03.2023 JP 2023040871
(71) Applicant: OMRON Corporation, Kyoto 600-8530 (JP); Omron Healthcare Co., Ltd., Muko-shi, Kyoto 617-0002 (JP)
(72) Inventor: KIMURA ISHIDA, Yui, Kyoto-shi, Kyoto 600-8530 (JP); KOIZUMI, Masayuki, Kyoto-shi, Kyoto 600-8530 (JP); WANG, Danni, Kyoto-shi, Kyoto 600-8530 (JP); KUBO, Mitsuaki, Kyoto-shi, Kyoto 600-8530 (JP); KAWABATA, Yasuhiro, Muko-shi, Kyoto 617-0002 (JP); FUJII, Kenji, Muko-shi, Kyoto 617-0002 (JP); MATSUMURA, Naomi, Muko-shi, Kyoto 617-0002 (JP); FUKUNAGA, Seiji, Muko-shi, Kyoto 617-0002 (JP); YOSHIDA, Takuya, Kyoto-shi, Kyoto 600-8234 (JP)
(74) Representative: Isarpatent
(86) International application number: PCT/JP2024/006330
(87) International publication number: WO 2024/190341

(57) **Abstract**

A biological signal measurement device includes a dry-type electrode; a member configured to fix the electrode in a state in which the electrode is pressed against a living body; and a control body configured to measure a biological signal by the electrode. The control body includes: an impedance measurement unit configured to measure impedance between the electrode and the living body, and an attachment state determination unit configured to determine whether an attachment state of the electrode is good or poor, based on an impedance value at a time when an elapsed time from attachment of the electrode to the living body by the member reaches a predetermined time.

## Description

### TECHNICAL FIELD

The present invention relates to a biological signal measurement device for measuring a biological signal by an electrode fixed to a surface of the skin.

### BACKGROUND ART

A technique for fixing an electrode to the surface of the skin of a living body and measuring a biological signal such as an electrocardiogram or electromyogram is known, and such a technique is applied to various measurement devices. In this type of device, if a contact state between the electrode and the surface of the skin is not appropriate, the measurement accuracy may decrease or the measurement itself may become impossible.

Patent Literature 1 proposes an idea of determining whether an attachment state of each electrode is good or poor, in a myoelectric measurement device of a type in which an adhesive electrode is attached to the surface of the skin, by measuring an electrical impedance value between two electrodes or a resistance value between the electrodes.

### CITATION LIST

### PATENT LITERATURE

Patent Literature 1: JP 2007-195813 A

### SUMMARY OF INVENTION

### TECHNICAL PROBLEM

The present inventors have been developing a type of measurement device in which an electrode is fixed to the surface of the skin by wrapping a band in which a dry-type electrode is embedded (hereinafter, also referred to as "electrode band") around an arm or a leg. By adopting such a fixing structure of the dry-type electrode, there are advantages in that the measurement can be easily performed because the patient himself/herself can easily attach and detach the device, and long-term measurement can be easily performed while wearing the device because there is no skin rash or the like. However, on the other hand, if the patient himself/herself is allowed to attach and detach the device, it is expected that the frequency of attachment failure will increase. Therefore, from the perspective of improving the reliability and stability of measurement, it is desirable to provide a mechanism that automatically determines the attachment state of the dry-type electrode and notifies the patient when attachment failure occurs.

The present inventors have experimentally fabricated electrode bands and conducted repeated attachment tests, and have found that there are two causes of attachment failure of the electrode, which are improper contact of the electrode with the surface of the skin (physical connection failure) and insufficient wetting between the electrode and the surface of the skin. Furthermore, it has been found that in many cases, insufficient wetting is resolved by perspiration after a certain period of time has elapsed since the electrode was attached, and a certain number of patients do not have sufficient wetting between the electrode and the skin even after a certain period of time has elapsed.

In the device of Patent Literature 1, a wet-type electrode that is attached to the surface of the skin is used, and thus attachment failure due to insufficient wetting is not assumed. Therefore, when the determination method of Patent Literature 1 is simply applied to a dry-type electrode device, it is impossible to distinguish whether the cause is insufficient wetting or physical connection failure, and a determination result of attachment failure is uniformly output. However, when the cause is insufficient wetting, it is highly likely to be resolved over time, and therefore it is not appropriate to immediately determine an attachment failure. Rather, in the case of insufficient wetting, the patient is misguided because the cause is not resolved even when the electrode is re-attached.

The present invention has been made in view of the above situations, and an object thereof is to provide a technique for appropriately determining whether an attachment state of an electrode is good or poor, and for appropriately notifying a user of a determination result in a biological signal measurement device employing a dry-type electrode.

### SOLUTION TO PROBLEM

The present disclosure includes a biological signal measurement device including a dry-type electrode, a member configured to fix the electrode in a state in which the electrode is pressed against a living body, and a control body configured to measure a biological signal by the electrode, in which the control body includes an impedance measurement unit configured to measure impedance between the electrode and the living body, and an attachment state determination unit configured to determine whether an attachment state of the electrode is good or poor, based on an impedance value at a time when an elapsed time from attachment of the electrode to the living body by the member reaches a predetermined time.

The predetermined time may be set to a time of five minutes or more.

The dry-type electrode may include a plurality of electrodes, and the attachment state determination unit may determine the attachment state to be poor when an impedance value of at least one electrode of the plurality of electrodes is equal to or greater than a predetermined threshold value at a time when the elapsed time reaches the predetermined time.

The attachment state determination unit may determine the attachment state to be poor before the elapsed time reaches the predetermined time, when an impedance value of the electrode or a temporal change in impedance before the elapsed time reaches the predetermined time satisfies a predetermined criterion.

The dry-type electrode may include a plurality of electrodes, and the predetermined criterion may be a fact that all impedance values of the plurality of electrodes are equal to or greater than a predetermined upper limit value.

The dry-type electrode may include a plurality of electrodes, and the predetermined criterion may be a fact that a difference, a ratio, or a degree of separation between impedance values of some electrodes of the plurality of electrodes and impedance values of the other electrodes is equal to or greater than a predetermined value.

The predetermined criterion may be a fact that an increase in value appears in a temporal change in impedance.

The predetermined criterion may be a fact that a slope of decrease in impedance is smaller than a predetermined slope.

A notification unit configured to notify a user when the attachment state determination unit determines the attachment state to be poor may be further included.

The attachment state determination unit may identify a cause of a poor attachment state, based on an impedance value or a temporal change in impedance, and the notification unit may vary a notification to the user in accordance with the cause of the poor attachment state.

The control body may include an operation unit that is operated by a user after the user attaches the electrode, and may start counting the elapsed time when the user operates the operation unit.

The biological signal may be an electrocardiogram (ECG) signal.

The member may be a band provided with the electrode.

The band may be attached to an upper arm of the living body.

The present disclosure includes a method for controlling a biological signal measurement device configured to measure a biological signal by a dry-type electrode, the method including counting an elapsed time from attachment of the electrode to a living body, measuring impedance between the electrode and the living body, and determining whether an attachment state of the electrode is good or poor, based on an impedance value at a time when the elapsed time reaches a predetermined time.

The present invention may be regarded as a biological signal measurement device including at least a part of the above configuration, or may be regarded as an electrocardiogram measurement device that measures an ECG signal as a biological signal. Alternatively, the present invention may be regarded as an attachment state determination device that can be mounted on a biological signal measurement device. Further, the present invention can be regarded as an attachment state determination method including at least a part of the above processing, a method for controlling a biological signal measurement device, or a program for implementing such a method and a non-transitory recording medium on which the program is recorded. Note that the present invention can be configured by combining each of the above-described configurations and processes to the extent possible.

### ADVANTAGEOUS EFFECTS OF INVENTION

According to the present invention, it is possible to appropriately determine whether an attachment state of an electrode is good or poor, and to appropriately notify a user of a determination result in a biological signal measurement device employing a dry-type electrode.

### BRIEF DESCRIPTION OF THE DRAWINGS

[FIG. 1] FIG. 1 is a diagram illustrating a state in which a biological signal measurement device is worn on an upper arm.
[FIG. 2] FIG. 2 is a plan view of the biological signal measurement device.
[FIG. 3] FIG. 3 is a perspective view of the biological signal measurement device.
[FIG. 4] FIG. 4 is a block diagram illustrating a functional configuration of a control body.
[FIG. 5] FIG. 5 is a flowchart illustrating a flow of an attachment state determination process in a first embodiment.
[FIG. 6] FIG. 6 is a flowchart illustrating a flow of an attachment state determination process in a second embodiment.
[FIG. 7] FIG. 7 is a diagram illustrating an example of a temporal change in an impedance value.
[FIG. 8] FIG. 8 is a flowchart illustrating a flow of an attachment state determination process in a third embodiment.
[FIG. 9] FIG. 9 is a diagram illustrating an example of a criterion used when determining an attachment state in the third embodiment.

### DESCRIPTION OF EMBODIMENTS

### <Application Example>

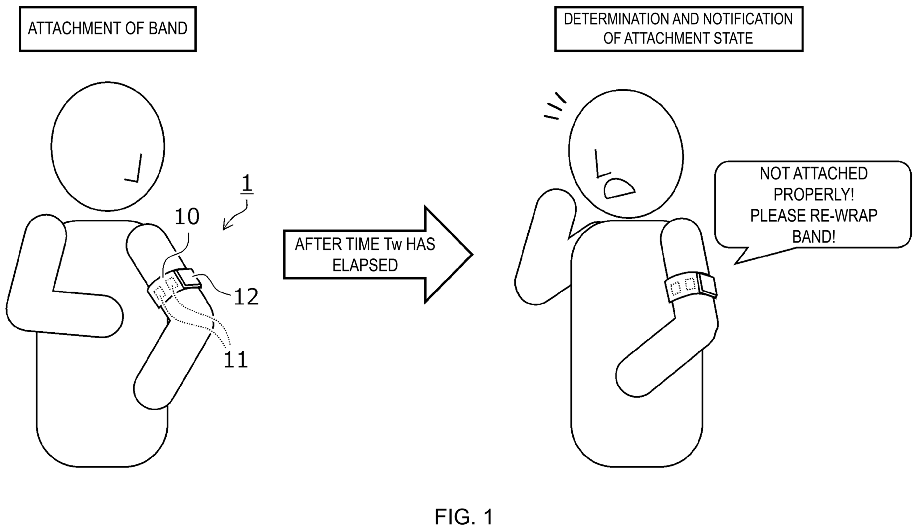

An application example of the present invention will be described with reference to FIG. 1.

A biological signal measurement device 1 is a portable measurement device that is used while being worn on a living body. The biological signal measurement device 1 includes, as main components, a band 10 provided with one or more dry-type electrodes 11, and a control body 12 that measures a biological signal by the electrodes 11.

The biological signal to be measured is also called a bioelectric signal or a biopotential, and is an electric signal generated in accordance with the activity of a living body. Examples include an electrocardiogram signal (a minute electrical signal associated with a heart rate), a myoelectric signal (a minute electrical signal associated with muscle activity), an electroencephalogram signal (a minute electrical signal associated with brain activity), and the like. Examples of measurement sites where the band 10 can be attached include upper limbs (upper arms, forearms, wrists, hands, and fingers), lower limbs (thighs, lower legs, ankles, feet, and toes), head, neck, chest, abdomen, and earlobe, and a measurement site is appropriately selected depending on the type of biological signal to be measured, a measurement algorithm, and the like.

At the time of measurement, a user himself/herself wears the device 1 by wrapping the band 10 around the measurement site, and then starts measurement by pressing a button of the control body 12 or the like. At this time, if there is a physical connection failure such as the electrode 11 being lifted from the surface of the skin due to poor wrapping or tightening of the band 10, correct measurement cannot be performed. Therefore, in such a case, it is necessary to notify the user so that a measure such as re-attaching the band 10 can be taken. However, even when the physical connection between the electrodes 11 and the surface of the skin is good, the electrical connection between the electrodes 11 and the surface of the skin may be poor due to insufficient wetting. Therefore, simply evaluating whether the electrical connection between the electrodes 11 and the surface of the skin is good or poor does not allow distinguishing between a physical connection failure and insufficient wetting, and does not allow providing the user with appropriate guidance.

Therefore, the biological signal measurement device 1 determines whether the attachment state of the electrodes 11 is good or poor, based on a contact impedance between the electrodes 11 and the surface of the skin at a time when an elapsed time from the attachment of the electrodes 11 reaches a predetermined time Tw. That is, whether the attachment state is good or poor is determined not immediately after the attachment of the electrodes 11, but after intentionally waiting for the predetermined time Tw. By providing such a waiting time, wetting between the electrodes 11 and the surface of the skin is expected to occur due to perspiration, and thus it becomes possible to determine whether there is a physical connection failure (that is, whether it is necessary to re-attach the electrodes 11) with high accuracy.

The predetermined time (waiting time) Tw may be set to a time of 5 minutes or more and 30 minutes or less. This is because, when such a time period elapses in a state in which the electrodes 11 are attached, a sufficient wetting state (that is, a state in which electrical connection between the electrodes 11 and the surface of the skin is maintained to a degree sufficient to allow measurement and evaluation of the contact impedance) is achieved for most users.

Hereinafter, as an embodiment of the present invention, a specific configuration example in a case where the present invention is applied to an upper arm electrocardiographic device will be described.

### <First Embodiment>

### (Device Configuration)

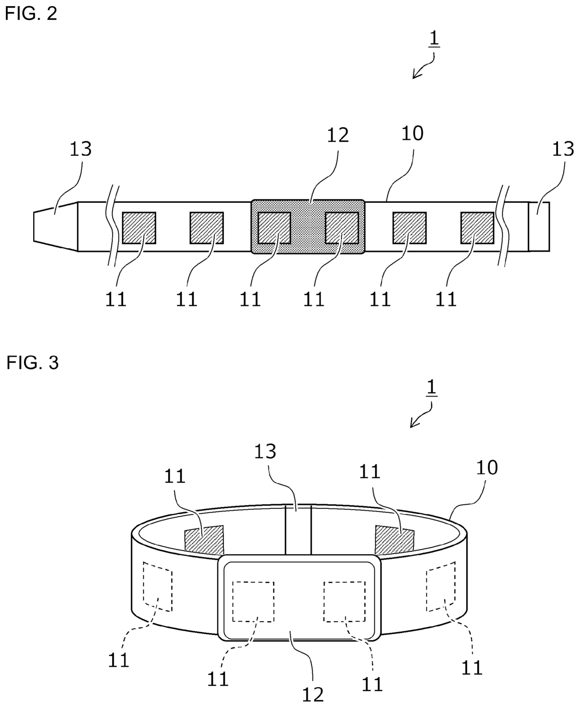

An embodiment of the present invention will be described with reference to FIGS. 1 to 3. FIG. 1 is a diagram illustrating a state in which the biological signal measurement device 1 is worn on an upper arm, FIG. 2 is a plan view of the biological signal measurement device 1, and FIG. 3 is a perspective view of the biological signal measurement device 1.

The biological signal measurement device 1 of the present embodiment is an upper arm electrocardiographic device that is worn on an upper arm (preferably, a left upper arm close to the heart) of a user and is used to measure an electrocardiogram (ECG) signal as a biological signal.

The biological signal measurement device 1 includes, as main components, a band 10, a plurality of electrodes 11 fixed to the band 10, and a control body 12 fixed to the band 10.

The band 10 is a member (fixing member) for fixing the electrodes 11 in a state in which the electrodes are pressed against the living body. In the present embodiment, a belt-shaped band 10 made of a flexible and pliable material (for example, chemical fiber, silicon, leather, or the like) is used. A fixing mechanism 13 is provided at a longitudinal end portion of the band 10. As illustrated in FIGS. 1 and 3, the biological signal measurement device 1 can be worn on the upper arm by forming the band 10 into a loop shape and fastening the band with the fixing mechanism 13. The fixing mechanism 13 may be any mechanism such as a hook-and-loop fastener, a hook, a connector, a button, or a magnet.

The plurality of electrodes 11 (also referred to as an electrode array) are embedded and fixed in the band 10 such that a contact surface with the living body is exposed to an inner side (living body side) of the band 10. The plurality of electrodes 11 are arranged in a line at equal intervals in the longitudinal direction of the band 10. Thus, when the band 10 is wrapped around the arm, the electrodes 11 come into contact with different circumferential positions of the arm. The number of electrodes 11 is not particularly limited and can be appropriately designed. At least two electrodes 11 (a pair of electrodes) may be provided to measure an ECG signal, and three or more electrodes 11 may be provided to increase the reliability and robustness of measurement. In the present embodiment, a configuration in which six electrodes 11 (three electrode pairs) are provided is adopted.

The electrode 11 is a dry-type metal electrode. The wet-type electrode (gel electrode or the like) has problems such as a possibility of causing skin rash or itching when attached for a long time, and low durability and maintainability, whereas the dry-type electrode 11 does not have such problems. The biological signal measurement device 1 of the present embodiment is assumed to be worn continuously for a long time and to monitor the ECG signal for 24 hours, and thus the electrodes 11 are preferably dry-type electrodes.

The control body 12 is a processing unit that performs control and signal processing of the biological signal measurement device 1. The control body 12 has a structure in which a processor, a memory, a battery, and other circuits are mounted inside a case made of resin or metal, for example. The control body 12 may be provided with a physical switch and a display. Although not illustrated, the control body 12 and the plurality of electrodes 11 are connected via signal lines.

### (Control Body)

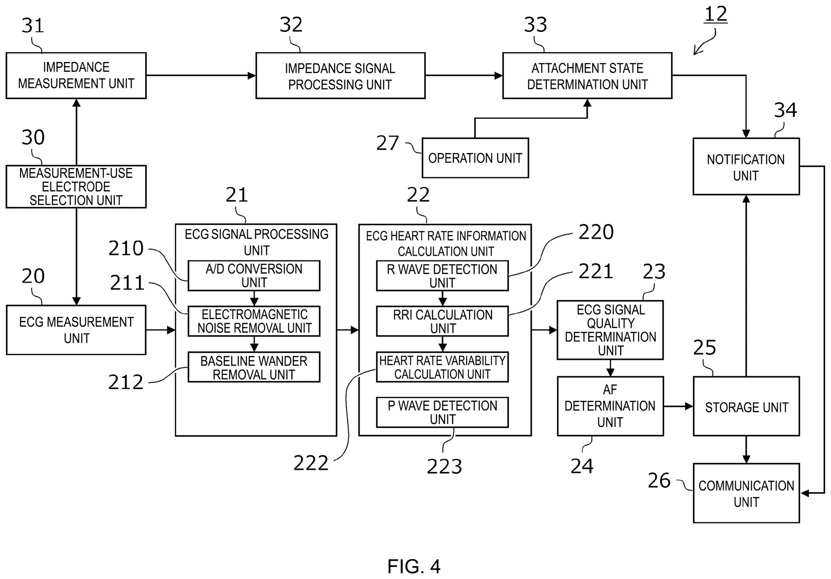

FIG. 4 is a block diagram illustrating an example of a functional configuration of the control body 12.

The control body 12 includes, as a configuration related to the measurement of the ECG signal, a measurement-use electrode selection unit 30, an ECG measurement unit 20, an ECG signal processing unit 21, an ECG heart rate information calculation unit 22, an ECG signal quality determination unit 23, an AF determination unit 24, a storage unit 25, a communication unit 26, and an operation unit 27. The measurement-use electrode selection unit 30 selects an electrode pair to be used for measurement of an ECG signal. The ECG measurement unit 20 is a circuit that amplifies a potential difference between the selected electrode pair by a differential amplifier and outputs the amplified potential difference as an ECG signal. The ECG signal processing unit 21 is a part that performs AD conversion and filtering processing of the ECG signal, and includes an AD conversion unit 210 that performs AD conversion of the ECG signal, an electromagnetic noise removal unit 211 that removes electromagnetic noise from the ECG signal to increase an SN ratio, and a baseline wander removal unit 212 that removes baseline wander (low-frequency wander) of the ECG signal. The ECG heart rate information calculation unit 22 is a part that extracts various types of heart rate information from the ECG signal, and includes an R wave detection unit 220, an RRI calculation unit 221, a heart rate variability calculation unit 222, and a P wave detection unit 223. The ECG signal quality determination unit 23 is a part that determines whether the quality of the ECG signal is good or poor (that is, whether accurate or reliable data suitable for diagnosis or the like can be measured). The AF determination unit 24 is a part that detects an occurrence of AF (Atrial Fibrillation) based on the heart rate information and calculates an indicator related to AF. The storage unit 25 is a nonvolatile memory that stores measured or calculated data. The communication unit 26 is a part that performs wireless data communication with an external device (for example, a smartphone of a user, another health device, a home server, or the like). The operation unit 27 is an input interface for a user to perform an input operation. The operation unit 27 may be a physical button or a touch panel display.

In addition, the control body 12 includes a measurement-use electrode selection unit 30, an impedance measurement unit 31, an impedance signal processing unit 32, an attachment state determination unit 33, and a notification unit 34, as components related to automatic determination of the attachment state of the electrodes 11. The measurement-use electrode selection unit 30 selects an electrode pair to be used for impedance measurement. The impedance measurement unit 31 is a circuit that measures a contact impedance between the electrodes 11 and the surface of the skin by causing a measurement current to flow between the selected electrode pair. In the present embodiment, a sinusoidal alternating current of approximately 10 Hz, which is close to an ECG signal, is used as the measurement current. The impedance signal processing unit 32 is a part that performs AD conversion or the like of the measured signal to obtain an impedance value (kΩ). The attachment state determination unit 33 is a part that determines whether the attachment state of the electrodes 11 is good or poor, based on the measured impedance value. The notification unit 34 is a part that performs notification to the user in accordance with a determination result of the attachment state determination unit 33.

### (Attachment State Determination Process)

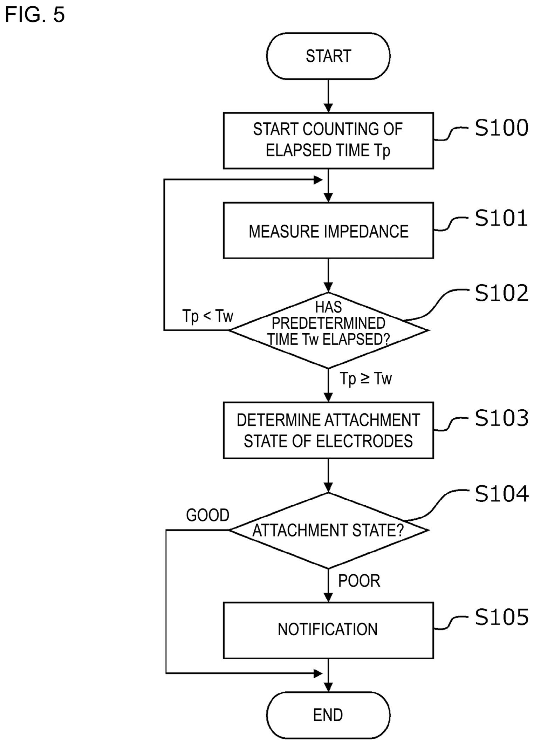

FIG. 5 illustrates a flow of an attachment state determination process in a first embodiment.

When the user wraps the band 10 around the upper arm, fastens the band 10 with the fixing mechanism 13, and then performs an operation of instructing the start of measurement with the operation unit 27, the processor of the control body 12 starts the determination process of FIG. 5. Note that although the ECG signal measurement process is also executed in parallel with the determination process in FIG. 5, the details of the measurement process are omitted in this specification because the measurement process is not directly related to the features of the present invention.

When the attachment state determination unit 33 of the control body 12 detects that the user performs an operation of instructing the start of measurement, the attachment state determination unit 33 determines that the user has attached the electrodes 11, and starts counting of the "elapsed time Tp from the attachment of the electrodes 11" (step S100). Note that, in the present embodiment, the user operation is used as a trigger for counting the elapsed time Tp, but the counting of the elapsed time Tp may be started by detecting the attachment of the band 10 or the electrodes 11 to the arm by an optical/electrical/magnetic/physical sensor.

In step S101, impedance is measured. Specifically, the measurement-use electrode selection unit 30 selects a pair of electrodes 11 to be used for measurement, the impedance measurement unit 31 measures a contact impedance between the selected electrodes 11, and the impedance signal processing unit 32 calculates a value of the contact impedance and stores the number n of the electrode 11, the elapsed time Tp, and the impedance value Zn as a set in the storage unit 25 (n = 1, 2,..., 6 when the number of electrodes 11 is six). By executing this measurement process for each of the plurality of electrodes 11, the impedance value of each of the electrodes 11 is recorded in the storage unit 25.

In step S102, the attachment state determination unit 33 determines whether the elapsed time Tp from the time of measurement in step S101 has reached the predetermined time Tw. When the elapsed time Tp has not reached the predetermined time Tw (Tp < Tw), the process returns to step S101. By such control, the impedance measurement is repeated at constant time intervals Ti until the elapsed time Tp reaches the predetermined time Tw. In the present embodiment, for example, Tw is set to 10 minutes, and Ti is set to 1 minute.

After the elapsed time Tp reaches the predetermined time Tw (Tp ≥ Tw), the attachment state determination unit 33 determines whether the attachment state of the electrodes 11 is good or poor, based on the impedance value Zn at that time (step S103). Specifically, the attachment state determination unit 33 determines the attachment state to be "good" when the impedance values Zn of all the electrodes 11 are below a predetermined threshold value Zth, and determines the attachment state to be "poor" when the impedance value Zn of at least any one of the electrodes 11 is equal to or higher than the threshold value Zth. In the present embodiment, for example, Zth is set to 100 kΩ.

When the attachment state is determined to be poor (step S104), the notification unit 34 notifies the user and prompts the user to re-wrap the band 10 (step S105). The method of notification is not particularly limited. For example, it is conceivable to issue a warning sound, to output a voice message prompting the user to re-wrap the band 10, to display a message on a display, to perform notification by vibration or light, to transfer a message to an external device (such as a smartphone possessed by the user), or the like.

In the determination process of the present embodiment, the contact state between the electrodes 11 and the surface of the skin is evaluated based on the contact impedance not immediately after the attachment of the electrodes 11 but after a predetermined time has elapsed. By waiting until wetting between the electrodes 11 and the surface of the skin is sufficiently increased by perspiration and then measuring and evaluating the impedance, a measurement failure due to insufficient wetting can be excluded as much as possible. Accordingly, a physical connection failure such as lift-off of the electrode 11 (that is, a state in which the band 10 needs to be re-wrapped) can be determined with high accuracy.

### <Second Embodiment>

It is considered that, in most cases, the insufficient wetting can be resolved by providing the waiting time of the predetermined time Tw, as in the first embodiment. However, there may be a certain number of individuals for whom sufficient wetting between the electrodes 11 and the skin is not achieved even after a sufficient time has elapsed. It may also depend on the environment (temperature, humidity, etc.) at the site. If the cause is the insufficient wetting, the situation cannot be improved by re-wrapping the band 10, and it becomes necessary to take a measure to increase the wetting between the electrodes 11 and the surface of the skin, such as applying cream or lotion to the skin or moistening the electrode. Therefore, in the second embodiment, after the attachment state is determined to be poor by the method of the first embodiment, it is identified whether a cause of the poor attachment state is insufficient wetting or physical connection failure.

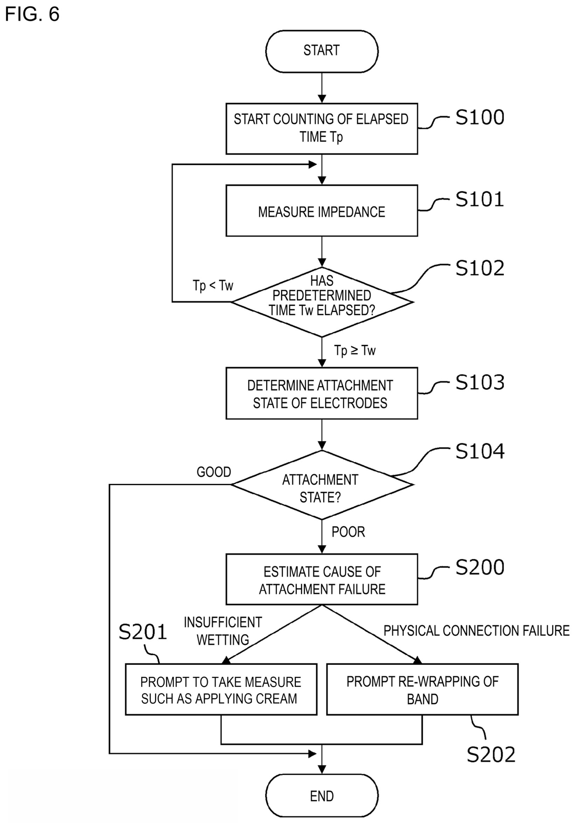

FIG. 6 illustrates a flow of an attachment state determination process in the second embodiment. The same step numbers are given to the same parts as those in the determination process of the first embodiment. Hereinafter, the processing different from that of the first embodiment will be mainly described.

When the attachment state is determined to be poor (step S104), the attachment state determination unit 33 estimates whether the cause of the attachment failure is physical connection failure such as lift-off of the electrode 11 or insufficient wetting between the electrodes 11 and the skin, based on the impedance value Zn of each of the electrodes 11 (step S200). Specifically, the attachment state determination unit 33 determines that "insufficient wetting is the cause" when the impedance values Zn of all the electrodes 11 are equal to or greater than the threshold value Zth, and determines that "physical connection failure is the cause" in other cases. Even when the band 10 is wrapped in a poor manner, it is unlikely that a physical connection failure occurs in all the electrodes 11, and at least some of the electrodes 11 are in close contact with the skin. Therefore, it is reasonable to consider that insufficient wetting is the cause when none of the electrodes 11 has an impedance value Zn lower than the threshold value Zth, even after the predetermined time Tw has elapsed.

The notification unit 34 varies a notification to the user depending on whether the cause of the poor attachment state is insufficient wetting between the electrodes 11 and the surface of the skin or improper contact between the electrodes 11 and the surface of the skin. For example, when it is determined in step S200 that the cause is insufficient wetting, the notification unit 34 prompts the user to take a measure to increase wetting, such as applying cream (step S201). On the other hand, when it is determined in step S200 that the cause is physical connection failure, the notification unit 34 prompts the user to re-wrap the band 10 (step S202).

According to the determination process of the present embodiment, in addition to the same operational effects as those of the first embodiment, there is an advantage in that it is possible to provide a measurement device with excellent usability, which can guide the user to take an appropriate measure when insufficient wetting is not resolved due to the user's constitution or the environment.

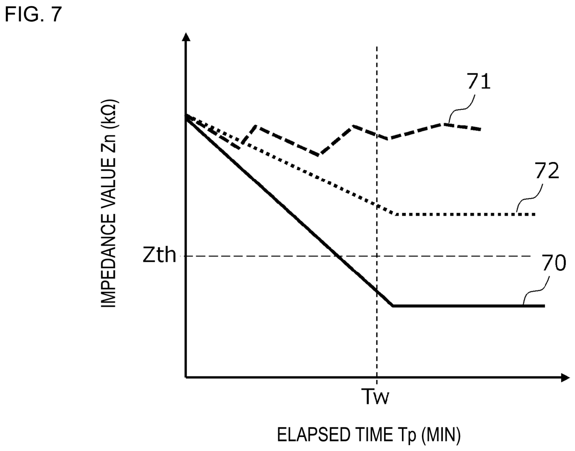

Note that the cause estimation in step S200 may be performed by another method. FIG. 7 illustrates an example of a temporal change in the impedance value Zn. The horizontal axis represents the elapsed time Tp (minutes), and the vertical axis represents the impedance value Zn (kΩ). The three graphs respectively illustrate a normal case 70, a case 71 where there is a physical connection failure such as lift-off of the electrode 11, and a case 72 where insufficient wetting is not resolved. As can be seen from FIG. 7, in the normal case 70, even when the impedance value Zn is initially high due to insufficient wetting, the wetting increases over time, and the impedance value Zn gradually decreases and falls below the threshold value Zth at the time of the predetermined time Tw. However, in the case 71 where there is a physical connection failure, the contact state between the electrode 11 and the surface of the skin is not stable, and therefore the impedance value Zn changes unstably and irregularly, such as rising and falling. In the case 72 where insufficient wetting is not resolved, the electrode 11 and the surface of the skin are in contact with each other, and therefore a behavior is exhibited in which the impedance value Zn gradually decreases over time, but the slope is small, and the impedance value Zn does not fall below the threshold Zth for an extended period. Therefore, for example, the attachment state determination unit 33 may check the temporal change in the impedance value Zn from immediately after the attachment of the band 10 to the predetermined time Tw, and determine that "the cause is a physical connection failure" when the impedance value Zn increases in the middle of the change, and determine that "the cause is insufficient wetting" when the impedance value Zn monotonically decreases.

### <Third Embodiment>

In the first and second embodiments, the determination of the attachment state of the electrodes 11 is performed after the predetermined time Tw has elapsed. However, when the attachment failure can be clearly determined before the predetermined time Tw has elapsed, the user may be notified at that time.

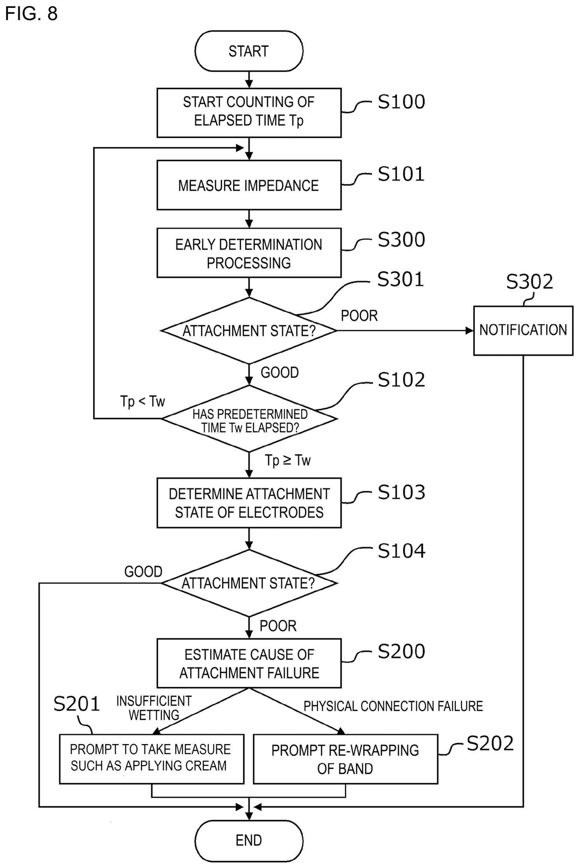

FIG. 8 illustrates a flow of an attachment state determination process in a third embodiment. The same step numbers are given to the same parts as those in the determination processes of the first and second embodiments. Hereinafter, the processing different from that of the first and second embodiments will be mainly described.

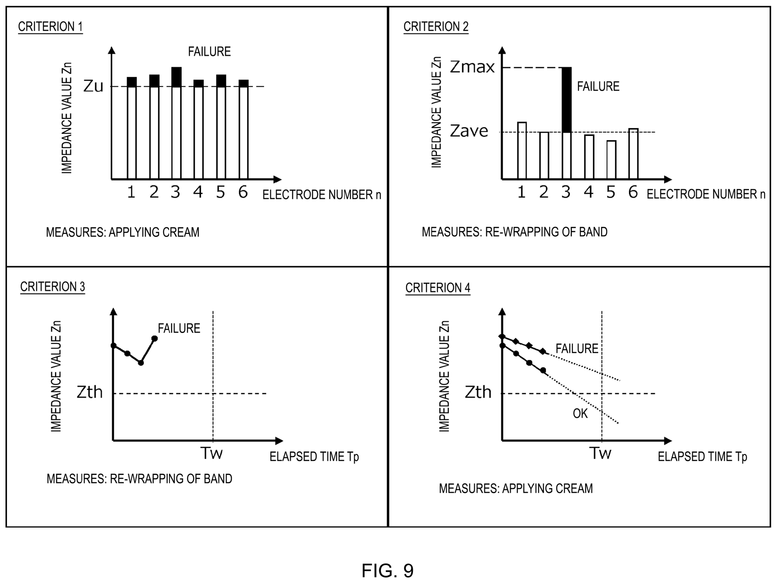

After the impedance measurement is performed in step S101, the attachment state determination unit 33 determines whether the attachment state of the electrodes 11 is good or poor, based on the impedance value Zn from immediately after the attachment of the electrodes 11 to the present time, in step S300. The determination processing of the step S300 is referred to as "early determination processing" in order to distinguish it from the determination processing of the step S103. When the attachment failure is determined in the early determination processing (step S301), the notification is immediately performed and the process is terminated (step S302). In the early determination process, it is evaluated whether the impedance value Zn or the temporal change thereof satisfies a predetermined criterion. FIG. 9 illustrates an example of the predetermined criterion.
(1) The criterion 1 is such that all the impedance values Zn of the plurality of electrodes 11 are equal to or greater than a predetermined upper limit value Zu. The upper limit value Zu indicates a state in which the skin is so extremely dry that sufficient wetting cannot be achieved even after the predetermined time Tw has elapsed. In the present embodiment, for example, Zu is set to 900 kΩ. For example, in the first measurement immediately after the attachment of the electrodes 11, when the impedance values Zn of all the electrodes 11 are equal to or greater than Zu, the attachment state determination unit 33 immediately determines the attachment failure, and the notification unit 34 prompts the user to take a measure such as applying cream.
(2) The criterion 2 is such that the impedance values Zn of some of the plurality of electrodes 11 are significantly higher than the impedance values Zn of the other electrodes 11. When the band 10 is wrapped in a poor manner, the number of electrodes 11 that cause a physical connection failure is about one or two. Therefore, when the impedance values Zn of the plurality of electrodes 11 are compared, only the impedance values Zn of some of the electrodes 11 in which the connection failure occurs are significantly higher than those of the other electrodes 11. When such a situation is observed, the attachment failure is immediately determined, and the user is prompted to re-wrap the band 10. Note that the determination as to whether the impedance values Zn of some of the electrodes 11 are significantly high may be performed in any manner. For example, when a difference between a maximum value Zmax among the impedance values Zn of the plurality of electrodes 11 and an average value Zave of the other impedance values Zn is equal to or greater than a predetermined value, "significantly high" may be determined. Alternatively, when a ratio of the maximum value Zmax to the average value Zave is equal to or greater than a predetermined value, "significantly high" may be determined. Alternatively, the plurality of electrodes 11 may be classified into two classes, namely, a first class and a second class, in accordance with the magnitude of the impedance value Zn, and when a difference or ratio between a representative value (for example, a maximum value, an average value, or the like) of the impedance values Zn of the first class and a representative value (for example, a maximum value, an average value, or the like) of the impedance values Zn of the second class is equal to or greater than a predetermined value, "significantly high" may be determined. Alternatively, when a degree of separation between the first class and the second class (such as a variance between the classes) is equal to or greater than a predetermined value, "significantly high" may be determined.
(3) The criterion 3 is such that an increase in value appears in the temporal change in the impedance value Zn. As described in the second embodiment, when the electrode 11 is correctly in contact with the skin, the impedance value Zn monotonically decreases. Therefore, when an increase in the impedance value Zn occurs in any of the plurality of electrodes 11, it means that a physical connection failure occurs in the electrode 11, and thus the attachment failure is immediately determined and the user is prompted to re-wrap the band 10.
(4) The criterion 4 is such that a slope of decrease in the impedance value Zn is smaller than a predetermined slope. When the electrode 11 is correctly in contact with the skin, the impedance value Zn monotonically decreases. A decrease curve in this case can be approximated by a linear expression in a simple manner. Therefore, for example, the slope of the decrease curve is approximately calculated at the stage where the impedance is measured at three to four points, and it is possible to predict to what extent the impedance value Zn decreases after the predetermined time Tw elapses. Here, if the slope of the decrease curve is too small and the impedance value Zn cannot be expected to fall below the threshold value Zth even after the predetermined time Tw has elapsed, the attachment failure is immediately determined and the user is prompted to take a measure such as applying cream.

By combining the above-described determination processing, when the attachment failure can be clearly determined, it is possible to prompt the user to take a measure before the elapsed time Tp reaches the predetermined time Tw. For example, in the case of the criteria 1 and 2, the determination can be made even immediately after the attachment of the electrodes 11, in the case of the criterion 3, the determination can be made at the time when the impedance rises, and in the case of the criterion 4, the determination can be made at the time when the measurement values are accumulated to the extent that the decrease curve of the impedance can be predicted. Therefore, according to the present embodiment, the convenience of the device can be further enhanced.

### <Others>

The embodiments described above are merely illustrative of configuration examples of the present invention. The present invention is not limited to the specific aspects described above, and various modifications are possible within the scope of the technical idea of the present invention. For example, the body part on which the device is worn may be other than the upper arm. In addition, as the fixing member for fixing the electrodes 11 in a state in which the electrodes are pressed against the living body, a band-shaped (belt-shaped) member that is wrapped around the measurement portion of the living body in a state in which the electrodes 11 are brought into contact, as in the above embodiments, an envelope-shaped member that covers and wraps the measurement portion of the living body, or a ring-shaped member may be used. Additionally, the fixing member may have elasticity or deformability in order to correspond to the size (diameter) of the measurement portion of the living body. Alternatively, when the fixing member is made of a non-elastic material, the fixing member may have a structure that allows adjustment of the length. The number of electrodes 11 may be one or more. The arrangement of the plurality of electrodes 11 is not necessarily one line, and the electrodes 11 may be arranged in a two-dimensional array. In addition, the electrodes 11 may be arranged at irregular intervals, instead of at equal intervals. The electrode 11 may be integrated with the fixing member (band 10), or may have a separate structure from the fixing member. The control body 12 need not be fixed to the band 10. For example, the control body 12 and the band 10 may be formed as separate structures, and the control body 12 and the band 10 (the electrode 11) may be connected to each other by a cable. The biological signal to be measured is not limited to the ECG signal, and may be, for example, a myoelectric signal or a brain wave signal.

The present specification includes the following disclosures.

### [Supplementary Note 1]

A biological signal measurement device (1) including:
a dry-type electrode (11);
a member (10) configured to fix the electrode (11) in a state in which the electrode (11) is pressed against a living body; and
a control body (12) configured to measure a biological signal by the electrode (11), in which
the control body (12) includes:
   an impedance measurement unit (31) configured to measure impedance between the electrode (11) and the living body, and
   an attachment state determination unit (33) configured to determine whether an attachment state of the electrode (11) is good or poor, based on an impedance value at a time when an elapsed time from attachment of the electrode (11) to the living body by the member (10) reaches a predetermined time.

### [Supplementary Note 2]

The biological signal measurement device (1) according to Supplementary Note 1, in which the predetermined time is set to a time of 5 minutes or more.

### [Supplementary Note 3]

The biological signal measurement device (1) according to Supplementary Note 1 or 2, in which
the dry-type electrode includes a plurality of electrodes(11), and
the attachment state determination unit (33) determines the attachment state to be poor when an impedance value of at least one electrode (11) of the plurality of electrodes (11) is equal to or greater than a predetermined threshold value at a time when the elapsed time reaches the predetermined time.

### [Supplementary Note 4]

The biological signal measurement device (1) according to any one of Supplementary Notes 1 to 3, in which
the attachment state determination unit (33) determines the attachment state to be poor before the elapsed time reaches the predetermined time, when an impedance value of the electrode (11) or a temporal change in impedance before the elapsed time reaches the predetermined time satisfies a predetermined criterion.

### [Supplementary Note 5]

The biological signal measurement device (1) according to Supplementary Note 4, in which
the dry-type electrode includes a plurality of electrodes (11), and
the predetermined criterion is a fact that all impedance values of the plurality of electrodes (11) are equal to or greater than a predetermined upper limit value.

### [Supplementary Note 6]

The biological signal measurement device (1) according to Supplementary Note 4 or 5, in which
the dry-type electrode includes a plurality of electrodes (11), and
the predetermined criterion is a fact that a difference, a ratio, or a degree of separation between impedance values of some electrodes (11) of the plurality of electrodes (11) and impedance values of the other electrodes (11) is equal to or greater than a predetermined value.

### [Supplementary Note 7]

The biological signal measurement device (1) according to any one of Supplementary Notes 4 to 6, in which the predetermined criterion is a fact that an increase in value appears in a temporal change in impedance.

### [Supplementary Note 8]

The biological signal measurement device (1) according to any one of Supplementary Notes 4 to 7, in which
the predetermined criterion is a fact that a slope of decrease in impedance is smaller than a predetermined slope.

### [Supplementary Note 9]

The biological signal measurement device (1) according to any one of Supplementary Notes 1 to 8, further including
a notification unit (34) configured to notify a user when the attachment state determination unit determines the attachment state to be poor.

### [Supplementary Note 10]

The biological signal measurement device (1) according to Supplementary Note 9, in which
the attachment state determination unit (33) identifies a cause of a poor attachment state, based on an impedance value or a temporal change in impedance, and
the notification unit (34) varies a notification to the user in accordance with the cause of the poor attachment state.

### [Supplementary Note 11]

The biological signal measurement device (1) according to any one of Supplementary Notes 1 to 10, in which
the control body (12)
includes an operation unit (27) that is operated by a user after the user attaches the electrode (11), and
starts counting of the elapsed time when the user operates the operation unit (27).

### [Supplementary Note 12]

The biological signal measurement device (1) according to any one of Supplementary Notes 1 to 11, in which
the biological signal is an electrocardiogram (ECG) signal.

### [Supplementary Note 13]

The biological signal measurement device (1) according to any one of Supplementary Notes 1 to 12, in which
the member is a band (10) provided with the electrode (11).

### [Supplementary Note 14]

The biological signal measurement device (1) according to Supplementary Note 13, in which
the band (10) is attached to an upper arm of the living body.

### [Supplementary Note 15]

A method for controlling a biological signal measurement device (1) configured to measure a biological signal by a dry-type electrode (11), the method including:
counting an elapsed time from attachment of the electrode (11) to a living body;
measuring impedance between the electrode (11) and the living body; and
determining whether an attachment state of the electrode (11) is good or poor, based on an impedance value at a time when the elapsed time reaches a predetermined time.

### REFERENCE SIGNS

1: Biological signal measurement device
10: Band
11: Electrode
12: Control body
13: Fixing mechanism

## Claims

1. A biological signal measurement device comprising:
a dry-type electrode;
a member configured to fix the electrode in a state in which the electrode is pressed against a living body; and
a control body configured to measure a biological signal by the electrode,
wherein
the control body includes:
an impedance measurement unit configured to measure impedance between the electrode and the living body, and
an attachment state determination unit configured to determine whether an attachment state of the electrode is good or poor, based on an impedance value at a time when an elapsed time from attachment of the electrode to the living body by the member reaches a predetermined time.

2. The biological signal measurement device according to claim 1, wherein the predetermined time is set to a time of 5 minutes or more.

3. The biological signal measurement device according to claim 1 or 2, wherein
the dry-type electrode includes a plurality of electrodes, and
the attachment state determination unit determines the attachment state to be poor when an impedance value of at least one electrode of the plurality of electrodes is equal to or greater than a predetermined threshold value at a time when the elapsed time reaches the predetermined time.

4. The biological signal measurement device according to any one of claims 1 to 3,
wherein
the attachment state determination unit determines the attachment state to be poor before the elapsed time reaches the predetermined time, when an impedance value of the electrode or a temporal change in impedance before the elapsed time reaches the predetermined time satisfies a predetermined criterion.

5. The biological signal measurement device according to claim 4, wherein
the dry-type electrode includes a plurality of electrodes, and
the predetermined criterion is a fact that all impedance values of the plurality of electrodes are equal to or greater than a predetermined upper limit value.

6. The biological signal measurement device according to claim 4 or 5, wherein
the dry-type electrode includes a plurality of electrodes, and
the predetermined criterion is a fact that a difference, a ratio, or a degree of separation between impedance values of some electrodes of the plurality of electrodes and impedance values of the other electrodes is equal to or greater than a predetermined value.

7. The biological signal measurement device according to any one of claims 4 to 6,
wherein the predetermined criterion is a fact that an increase in value appears in a temporal change in impedance.

8. The biological signal measurement device according to any one of claims 4 to 7,
wherein
the predetermined criterion is a fact that a slope of decrease in impedance is smaller than a predetermined slope.

9. The biological signal measurement device according to any one of claims 1 to 8,
further comprising
a notification unit configured to notify a user when the attachment state determination unit determines the attachment state to be poor.

10. The biological signal measurement device according to claim 9, wherein
the attachment state determination unit identifies a cause of a poor attachment state, based on an impedance value or a temporal change in impedance, and
the notification unit varies a notification to the user in accordance with the cause of the poor attachment state.

11. The biological signal measurement device according to any one of claims 1 to 10,
wherein
the control body
includes an operation unit that is operated by a user after the user attaches the electrode, and
starts counting of the elapsed time when the user operates the operation unit.

12. The biological signal measurement device according to any one of claims 1 to 11,
wherein
the biological signal is an electrocardiogram (ECG) signal.

13. The biological signal measurement device according to any one of claims 1 to 12,
wherein
the member is a band provided with the electrode.

14. The biological signal measurement device according to claim 13, wherein
the band is attached to an upper arm of the living body.

15. A method for controlling a biological signal measurement device configured to measure a biological signal by a dry-type electrode, the method comprising:
counting an elapsed time from attachment of the electrode to a living body,
measuring impedance between the electrode and the living body; and
determining whether an attachment state of the electrode is good or poor, based on an impedance value at a time when the elapsed time reaches a predetermined time.
